# EUROPEAN PATENT SPECIFICATION

(11) **EP 1 459 729 B1**
(45) Date of publication and mention of the grant of the patent: **10.12.2008**
(21) Application number: 04250859.8
(22) Date of filing: 18.02.2004
(51) Int. Cl.: A61K 8/73, A61K 8/81, A61Q 19/00

(54) **Skin or hair care cosmetic composition**
Hautpflegende oder haarpflegende kosmetische Zusammensetzung
Composition cosmétique pour le soin de la peau ou des cheveux

(30) Priority: 18.02.2003 JP 2003039487
(43) Date of publication of application: 22.09.2004
(73) Proprietor: Shin-Etsu Chemical Co., Ltd., Chiyoda-ku, Tokyo (JP)
(72) Inventor: Hayakawa, Kazuhisa Shin-Etsu Chemical Co., Ltd, Nakakubiki-gun Niigata-ken (JP)
(74) Representative: Stoner, Gerard Patrick

(56) References cited:
- EP-A- 1 132 076
- EP-A- 1 319 388
- EP-A- 1 340 485
- US-A- 5 199 954
- US-A- 5 420 104
- US-B1- 6 368 581

## Description

This invention relates to a skin care or hair care cosmetic composition.

### Background

In numerous cosmetic or personal care products, a variety of materials have been used for the purposes of fluidity improvement, thickening and emulsion stabilization, and for fixing the desired function to the skin or hair. Known materials include anionic derivatives of starch, xanthane gum and cellulose (e.g., carboxymethylated starch and cellulose, phosphoryled starch and cellulose, cationic cellulose and derivatives thereof), polyacrylic acid and the like. A recent progress is found in JP-A 10-511422 disclosing that a uniform ternary copolymer of vinyl pyrrolidone, a quaternary ammonium monomer and a hydrophobic monomer is effective in hair care compositions. Also, JP-A 10-511998 discloses that a substantially uniform copolymer of vinyl pyrrolidone with N-3,3-dimethylaminopropyl methacrylamide is effective in hair styling compositions.

As rheological improvers, thickeners and emulsion stabilizers, there are known polyacrylic acid salts, alginic acid salts, carrageenan, hyaluronic acid salts, carboxymethyl cellulose salts, trigluco-polysuccharides, carboxymethyldextran salts, cationic cellulose, cationic guar gum, carboxyvinyl polymer salts, xanthane gum, bentonite, magnesium aluminum silicate, methyl cellulose, hydroxypropyl methyl cellulose, hydroxyethyl cellulose, etc. Sweger et al. USP 5,482,704 or JP-A 8-40822 discloses a cosmetic composition comprising an amino-multicarboxylate modified starch derivative. JP-A 2000-297028 discloses that fatty acid-N-alkyl polyhydroxyalkylamides are useful in skin care compositions.

However, these conventional known materials or improvers, when used alone, fail to impart fully satisfactory functions with respect to rheological improvement, thickening, moisturizing protection, emulsion stabilization, and fixation of effective ingredients (e.g., oils and alcohols) to the skin or hair. It is thus proposed to use these improvers in combination. However, if cosmetic compositions prepared by combining these improvers are allowed to stand over a long period of time, the polymers combined will separate from each other or agglomerate together under certain temperature conditions, that is, the cosmetic compositions lack shelf stability. In particular, water-soluble alkyl celluloses, hydroxyalkyl alkyl celluloses and hydroxypropyl cellulose, whose solution in water thermally turns to gel when heated and back to aqueous solution when cooled, remain stable as dispersions in cosmetic base materials such as silicone oil (see K. Hayakawa, M. Kawaguchi, T. Kato, Langmuir, 13, 6069 (1997)) and are thus useful in providing flow and moisture retention properties to cosmetic base materials. If such a cellulose is used in combination with a synthetic polymer for the purpose of further improving flow or moisturizing protection, the poor compatibility between them causes the cosmetic base material to separate or partially agglomerate during long-term storage at elevated temperatures, despite a certain improvement made by the combined use with the synthetic polymer, so that the resulting cosmetic composition has undesirably poor stability.

It is an object that embodiments of the invention provide a skin care or hair care cosmetic composition which remains stable during shelf storage. Preferably, embodiments have improved flow, viscosity buildup, moisturising protection, emulsion stability and fixation of cosmetic ingredients such as oils and alcohols to the skin or hair as required for such cosmetic compositions.

It has been found that the above-discussed problem can be addressed by a composition comprising a nonionic cellulose ether and a copolymer of vinyl pyrrolidone with an amino-containing cationic compound.

The invention provides a cosmetic composition for skin care or hair care comprising (A) a nonionic cellulose ether selected from methyl cellulose, hydroxypropyl methyl cellulose and hydroxypropyl cellulose and (B) a copolymer of 1-ethenyl-2-pyrrolidone with N,N-dimethyl-N-(3-((2-methyl-1-oxo-2-propenyl)amino)propyl)l-dodecanaminium chloride and N-(3-(dimethylamino)propyl)2-methylpropenamide, and methods of making such compositions.

The skin care or hair care cosmetic composition of the invention remains stable during shelf storage at elevated temperatures, and is improved in flow, viscosity, moisturizing protection, and emulsion stability.

### DESCRIPTION OF OPTIONAL FEATURES AND PREFERRED EMBODIMENTS

Component (A) used in the skin care or hair care cosmetic composition of the invention is a nonionic cellulose ether. It is preferably a cellulose ether whose aqueous solution exhibits a thermal reversible gelation phenomenon. Preferred nonionic cellulose ethers include methyl cellulose, hydroxypropyl methyl cellulose and hydroxypropyl cellulose as prescribed in the Japanese Pharmacopoeia or Pharmaceutical Excipients Standards.
Especially preferred is hydroxypropyl methyl cellulose as prescribed in the Japanese Pharmacopoeia, 14th Edition, D-2208 because its thermal gelation temperature by heating is low and its stability when added to cosmetic compositions is high, especially at elevated temperatures. By contrast, anionic cellulose ethers such as carboxymethyl cellulose cannot be used because they form complexes with cationic polymers such as component (B) of the invention.

The nonionic cellulose ether which can be used herein is commercially available in powder form. If necessary, nonionic cellulose ether in granular form may be used which is prepared by fluidizing a nonionic cellulose ether powder, adding dropwise or spraying a powder-binding liquid containing a humectant such as a nonionic, anionic or cationic surfactant to the powder, granulating and drying. Such nonionic cellulose ether granules are fast soluble in cold water. The granular product is preferably fractionated such that the amount remaining on No. 30 (opening 500 µm) sieve described in the Japanese Pharmacopoeia, 14th Edition, B-1060 is up to 50% by weight and the amount passing through No. 140 (opening 106 µm) sieve described in the Japanese Pharmacopoeia, 14th Edition, B-1061 is up to 40% by weight. Dissolution may be performed by using this granulated, cold water-soluble, nonionic cellulose ether, and mixing a necessary amount thereof with a particulate polymer obtained through copolymerization of vinyl pyrrolidone with an amino-containing cationic compound. This may avoid environmental and other problems including dust generation during the dissolution operation and retarded dissolution.

Component (B) is a copolymer of 1-ethenyl-2-pyrrolidone with N,N-dimethyl-N-(3-((2-methyl-1-oxo-2-propenyl)amino propyl)1-dodecanaminium chloride and N-(3-(dimethylamino)propyl)2-methylpropenamide. When combined with the nonionic cellulose ether, this copolymer preferably improves the moisturising protection to the skin and hair and the flow and uniform dispersion of cosmetic ingredients.

Preferably the copolymers of vinyl pyrrolidone with an amino-containing cationic compound have a molecular weight of several thousand to several million, e.g. 3,000 to 3,000,000. The amount of the copolymer added is not particularly limited insofar as it is equal to or more than the minimum necessary amount to develop the desired function. Usually the amount of the copolymer added is 0.01 to 10% by weight, preferably 0.01 to 6% by weight based on the total weight of the cosmetic composition.

In the cosmetic composition, the nonionic cellulose ether (A) and the copolymer of vinyl pyrrolidone with an amino-containing cationic compound (B) are preferably included in a weight ratio A/B between 1/50 and 10/10, more preferably between 1/30 and 5/10. Preferably, within this range set for achieving the purposes of flow improvement, thickening and emulsion stabilization, the copolymer of vinyl pyrrolidone with an amino-containing cationic compound (B) imparts ionic property to the cosmetic ingredients and the skin or hair for providing stability and protective colloid action by way of electric repulsive forces, and the nonionic cellulose ether (A) serves to protect and stabilize the copolymer, improve the dispersion of the copolymer, and adsorb to other cosmetic ingredients in a uniform distribution. Both the components suitably cooperate in this way to address the problems. If the ratio A/B is less than 1/50, the protection and stabilization of component (B) by component (A) may become insufficient. If the ratio A/B is more than 10/10, the protection and stabilization of component (B) may be prohibited by excessive component (A).

The skin or hair care cosmetic composition of the invention includes the aforementioned components (A) and (B) and may optionally include well-known components depending on a particular application, usage or form of the cosmetic composition. For example, there may be added nonionic, cationic or anionic surfactants, minerals as oil component of emulsion compositions, animal and vegetable oils, fats, synthetic esters, aliphatic alcohols, higher aliphatic alcohols, alkylamines, waxes, so-called mineral fats, paraffin oil, petrolatum, ceresine and analogous oils, silicone fluids, PEG 300 distearate, sorbitan monolaurate, and triethanolamine stearate as well as humectants, tickeners, UV inhibitors, preservatives, pigments, dyes, colorants, flavors, fragrant agents, antiseptic agents, antifungal agents, antibacterial agents, etc. as long as they do not compromise the objects of the invention.

### EXAMPLES

Examples of the invention are given below by way of illustration and not by way of limitation.

### Example 1

| | |
|---|---|
| Ethyl alcohol | 10 wt% |
| Vinyl pyrrolidone derivative | 1 wt% |
| Nonionic cellulose ether | 0.025 wt% |
| Propylene glycol | 5.0 wt% |
| Polyoxyethylene sorbitan monolaurate | 1.2 wt% |
| Jasmine flavor | 0.2 wt% |
| Purified water | balance |
| Total | 100.0 wt% |

Purified water was heated at 80°C, after which hydroxypropyl methyl cellulose powder (90SH-4000 by Shin-Etsu Chemical Co., Ltd.) as the nonionic cellulose ether was admitted and dispersed therein. While cooling at 5°C, the remaining components including a copolymer of N,N-dimethyl-N-(3-((2-methyl-1-oxo-2-propenyl)amino)propyl) 1-dodecanaminium chloride and N-(3-(dimethylamino)propyl)-2-methylpropenamide with 1-ethenyl-2-pyrrolidone (trade name: STYLEZE® W-10, International Specialty Products) as the vinyl pyrrolidone derivative were admixed in the dispersion. The resulting solution was heated at 50°C and allowed to stand at the temperature for 30 days, finding that the solution remained stable. When the solution was applied to the skin, it flowed smoothly and kept the skin moist effectively.

### Example 2

| | |
|---|---|
| Vinyl pyrrolidone derivative | 5 wt% |
| Nonionic cellulose ether | 0.3 wt% |
| Triethanol amine | 0.5 wt% |
| Purified water | balance |
| Total | 100.0 wt% |

Purified water was heated at 80°C, after which hydroxypropyl methyl cellulose powder (90SH-100000 by Shin-Etsu Chemical Co., Ltd.) as the nonionic cellulose ether was admitted and dispersed therein. While cooling at 5°C, the remaining components including a copolymer of vinyl pyrrolidone with methacrylamidopropyl trimethylammonium chloride (trade name: GAFQUAT® HS-100, International Specialty Products) as the vinyl pyrrolidone derivative were admixed in the dispersion.

From the resulting composition, a hair conditioning/styling gel was prepared. It was heated at 50°C and allowed to stand at the temperature for 30 days, finding that the gel remained stable. When the gel was applied to the hair, it flowed smoothly and retained the shape of hair effectively.

## Claims

1. A cosmetic composition for skin care or hair care comprising
(A) a nonionic cellulose ether selected from methyl cellulose, hydroxypropyl methyl cellulose and hydroxypropyl cellulose,
(B) a copolymer of 1-ethenyl-2-pyrrolidone with N, N-dimethyl-N-(3-((2-methyl-1-oxo-2-propenyl)amino)propyl)1-dodecanaminium chloride and N-(3-(dimethyl amino)propyl)2-methylpropenamide.

2. The cosmetic composition of claim 1 wherein said nonionic cellulose ether is hydroxypropyl methyl cellulose.

3. The cosmetic composition of claim 1 or 2 wherein said nonionic cellulose ether is a granulated product obtained by granulating a non-ionic cellulose ether powder.

4. The cosmetic composition of any one of claims 1 to 3 wherein component (B) accounts for 0.01 to 10% by weight of the total weight of the composition and the weight ratio of component (A) to component (B) is between 1/50 and 10/10.

5. A method of preparing a cosmetic composition of any one of claims 1 to 4, including the step of mixing (i) a nonionic cellulose ether selected from methyl cellulose, hydroxypropyl methyl cellulose and hydroxypropyl cellulose with (ii) a copolymer of 1-ethenyl-2-pyrrolidone with N, N-dimethyl-N-(3-((2-methyl-1-oxo-2-propenyl)amino)propyl)1-dodecanaminium chloride and N-(3-(dimethyl amino)propyl)2-methylpropenamide.

6. The method of claim 5, including the step of granulating the nonionic cellulose ether prior to mixing.

## Patentansprüche

1. Kosmetische Zusammensetzung zur Haut- oder Haarpflege, umfassend:
(A) einen aus Methylcellulose, Hydroxypropylmethylcellulose und Hydroxypropylcellulose ausgewählten nicht-ionischen Celluloseether,
(B) ein Copolymer von 1-Ethenyl-2-pyrrolidon mit N,N-Dimethyl-N-(3-((2-methyl-1-oxo-2-propenyl)amino)propyl)-1-dodecanaminiumchlorid und N-(3-(Dimethylamino)propyl)-2-methylpropenamid.

2. Kosmetische Zusammensetzung nach Anspruch 1, worin der nicht-ionische Celluloseether Hydroxypropylmethylcellulose ist.

3. Kosmetische Zusammensetzung nach Anspruch 1 oder 2, worin der nicht-ionische Celluloseether ein granuliertes Produkt ist, das durch das Granulieren eines nicht-ionischen Celluloseetherpulvers erhalten wird.

4. Kosmetische Zusammensetzung nach einem der Ansprüche 1 bis 3, worin Komponente (B) 0,01 bis 10 Gew.-% des Gesamtgewichts der Zusammensetzung ausmacht und das Gewichtsverhältnis von Komponente (A) zu Komponente (B) zwischen 1/50 und 10/10 liegt.

5. Verfahren zur Herstellung einer kosmetischen Zusammensetzung nach einem der Ansprüche 1 bis 4, umfassend den Schritt des Mischens (i) eines aus Methylcellulose, Hydroxypropylmethylcellulose und Hydroxypropylcellulose ausgewählten nicht-ionischen Celluloseethers mit (ii) einem Copolymer von 1-Ethenyl-2-pyrrolidon mit N,N-Dimethyl-N-(3-((2-methyl-1-oxo-2-propenyl)amino)propyl)-1-dodecanaminiumchlorid und N-(3-(Dimethylamino)propyl)-2-methylpropenamid.

6. Verfahren nach Anspruch 5, umfassend den Schritt des Granulierens des nicht-ionischen Celluloseethers vor dem Mischen.

## Revendications

1. Composition cosmétique pour le soin de la peau ou le soin des cheveux comprenant
(A) un éther de cellulose non-ionique sélectionné parmi la méthyl cellulose, hydroxypropyl méthyl cellulose et hydroxypropyl cellulose,
(B) un copolymère de 1-éthényl-2-pyrrolidone avec N, N-diméthyl-N-(3-((2-méthyl-1-oxo-2-propényl)amino)propyl)1-dodécanaminium chlorure et N-(3-diméthyl amino)propyl)2-méthylpropénamide.

2. Composition cosmétique selon la revendication 1, où ledit éther de cellulose non-ionique est la hydroxypropyl méthyl cellulose.

3. Composition cosmétique selon la revendication 1 ou 2, où ledit éther de cellulose non-ionique est un produit granulé obtenu par granulation d'une poudre d'éther de cellulose non-ionique.

4. Composition cosmétique selon l'une quelconque des revendications 1 à 3, où le composant (B) représente 0,01 à 10% en poids du poids total de la composition, et le rapport pondéral du composant (A) au composant (B) est entre 1/50 et 10/10.

5. Procédé de préparation d'une composition cosmétique selon l'une quelconque des revendications 1 à 4, incluant l'étape consistant à mélanger (i) un éther de cellulose non-ionique sélectionné parmi la méthyl cellulose, hydroxypropyl méthyl cellulose et hydroxypropyl cellulose avec (ii) un copolymère de 1-éthényl-2-pyrrolidone avec N,N-diméthyl-N-(3-((2-méthyl-1-oxo-2-propényl)amino)propyl)1-dodécanaminium chlorure et N-(3-diméthyl amino)propyl)2-méthylpropénamide.

6. Procédé selon la revendication 5, incluant l'étape consistant à granuler l'éther de cellulose non-ionique avant le mélange.
